# EUROPEAN PATENT SPECIFICATION

(11) **EP 2 954 956 B1**
(45) Date of publication and mention of the grant of the patent: **16.10.2019**
(21) Application number: 15170700.7
(22) Date of filing: 04.06.2015
(51) Int. Cl.: B05B 12/00, B05B 13/04, B05B 13/02, A61M 25/00, B05B 12/16, B05B 12/08

(54) **APPARATUS FOR DOSING AND CONTROLLING A NEBULIZED SPRAY ON AN ELONGATED HOLLOW BODY PARTICULARLY FOR FOOD OR MEDICAL USE**
VORRICHTUNG ZUR DOSIERUNG UND STEUERUNG EINES ZERSTÄUBTEN SPRAYS AUF EINEM LÄNGLICHEN HOHLKÖRPER INSBESONDERE FÜR LEBENSMITTEL ODER FÜR MEDIZINISCHE VERWENDUNG
APPAREIL DE DOSAGE ET COMMANDE D'UN SPRAY NÉBULISÉ SUR UN CORPS CREUX ALLONGÉ EN PARTICULIER POUR USAGE ALIMENTAIRE OU MEDICAL

(30) Priority: 12.06.2014 IT PN20140032
(43) Date of publication of application: 16.12.2015
(73) Proprietor: Bucci Automations S.p.A., 48018 Faenza (RA) (IT)
(72) Inventor: Fant, Daniele, 32013 Longarone (BL) (IT)
(74) Representative: Gonella, Mario

(56) References cited:
- EP-A1- 2 319 628
- WO-A1-2010/006787
- WO-A2-2008/134575
- US-A1- 2013 230 639

## Description

### TECHNICAL FIELD OF THE INVENTION

. The present invention is relative to an apparatus for dosing and controlling a nebulized spray on an elongated hollow body, such as for example a station for metering and controlling a nebulized spray of solvent or adhesive on a pipe or tube of plastic material, in particular for medical or food use, for a subsequent permanent coupling with a second element, such as a connector, a closing plug or another pipe or tube.

### BACKGROUND OF THE INVENTION

. WO 2010/006787 describes a device and a method for applying a coating layer on the external surface of a hollow body suitable for medical use. The device comprises at least a nozzle and a nozzle support frame arranged so that the coating layer can be applied on the surface of the hollow body without there being any contact between the parts.

. One drawback of the prior solution known from WO 2010/006787 lies in the fact that particles of the substance nebulized or sprayed on the external surface and forming the coating layer can also penetrate inside the hollow body. Since it is a product designed to be used in the medical field, generally the hollow body holds within it medicines that could become contaminated by the substance of the coating layer, with the consequence that the medicine contained in the hollow body would no longer be in a sterile environment and could even be toxic for the patient.

. A further drawback lies in the absence of systems for controlling the discharge of the spray of nebulized substance from the nozzles. In fact, the exit orifice from the nozzle could become clogged, and thus prevent the discharge of the substance, and this would result in an imperfect application of the coating layer on the hollow body.

### SUMMARY OF THE INVENTION

. The main objective of the present invention is thus to devise an apparatus for dosing and controlling a nebulized spray on an elongated hollow body, in particular for medical or food uses, capable of overcoming the drawbacks of the known art.

. In the scope of the above objective, one purpose of the present invention is to devise an apparatus that is capable of spraying a jet of nebulized substance on the elongate hollow body in a manner to ensure that said substance does not contaminate the internal cavity of the hollow body.

. Another objective of the invention is to devise an apparatus capable of controlling the effective discharge of the spray of nebulized substance.

. One not secondary objective is to devise an apparatus for dosing and controlling a nebulized spray on an elongated hollow body that achieves the above purpose and objectives at competitive costs and that can be manufactured with the usual known machinery, plants and equipment.

. The above purpose and objectives, as well as others that will become more evident from the ensuing description, will be achieved by an apparatus for dosing and controlling a nebulized spray on an elongated hollow body as defined in claim 1.

### BRIEF DESCRIPTION OF THE FIGURES

. Further characteristics and advantages of the present invention will become more evident from the following description of a particular, but not exclusive embodiment illustrated purely by way of indicative but not limitative example with reference to the enclosed figures, wherein:
- figure 1 is a front perspective view of an apparatus for dosing and controlling a nebulized spray on an elongated hollow body according to the present invention, in an initial work cycle position;
- figure 2 is a cross-sectional perspective view along a median longitudinal axis of the apparatus of the preceding figure;
- figure 3 illustrates, in a front perspective view, the apparatus of the preceding figures in an operating position in the work cycle;
- figure 4 shows, in a cross-sectional rear perspective view, the apparatus in the operating position of figure 3;
- figure 5 shows, in a cross-sectional view, a second embodiment of the apparatus according to the present invention;
- figure 6 illustrates the embodiment of figure 5 according to a perspective view partially in cross section;
- figure 7 is a detail of the preceding figure, seen in perspective from a different viewing angle.

### DETAILED DESCRIPTION OF THE INVENTION

.With reference to the above-mentioned figures, number 1 indicates an apparatus for dosing and controlling a nebulized spray on an elongated hollow body, such as for example the spray of a nebulized solvent substance on a portion of the external surface of a tube of thermoplastic material for medical use to which must be subsequently connected by gluing a second element, such as a connector, a closing plug or another tube or hollow body. The chemical action of melting a superficial portion of the hollow body by the effect of the nebulized solvent makes it possible to bond the second element by glueing through fusion.

.The dosing and control apparatus according to the present invention can be advantageously integrated in a production line where it defines a workstation arranged upstream of the subsequent gluing station for the second element.

.The dosing and control apparatus 1 comprises at least one valve 2 provided with a nozzle 3 for ejecting the nebulized substance; the valve 2 is controlled by an electronic control unit that makes it possible to adjust the operating parameters, such as time and nebulization pressure.

.The valve 2 is supported on a carriage 4 that moves axially along an axis perpendicular to the axis of discharge of the valve 2 in the direction shown by the arrow A.

.The carriage 4 is also associated to a ring 5 provided with at least one hole 6 arranged so as to face the nozzle 3 of the valve 2, so that the nebulized flow is conveyed through the hole 6 into the cavity of the ring 5.

.The axial movement of the carriage 4 is perpendicular to the axis of discharge of the valve 2, as said above, and parallel to the axis of the ring 5.

.In the embodiment illustrated by way of example in the enclosed figures, there are three valves arranged circumferentially around the ring 5 at 120° from each other; each valve is controlled by an electronic control unit to regulate the nebulization parameters and the ring 5 is provided with a number of holes 6 that is equal to the number of valves, each hole being positioned facing the respective nozzle 3.

.The apparatus 1 is also provided with occluding means suitable to prevent the penetration of the nebulized substance inside the elongated hollow body, presented schematically in the enclosed figures by a hollow body 7, with means for centring the same hollow body 7 so that it can be properly sprayed by the flow of nebulized substance in correspondence of the portion desired, and with means for controlling the effective discharge of the spray of nebulized substance from the nozzle 3.

.In the embodiment illustrated in the enclosed figures, the occluding means and the centring means are integrated in a stem 8 arranged centrally and coaxially to the ring 5; the stem 8 is supported by a flange 9 fastened to the carriage 4 and is designed to slide axially 4 with respect to the ring 5 counteracting the action of elastic means 10, such as a preloaded spring.

.The terminal portion of the stem 8 turned toward the nozzle, or the nozzles, 3 is provided with an enlarged head 11 and a conical end 12 forming, respectively, the occluding means and the centring means, as will be better explained hereafter.

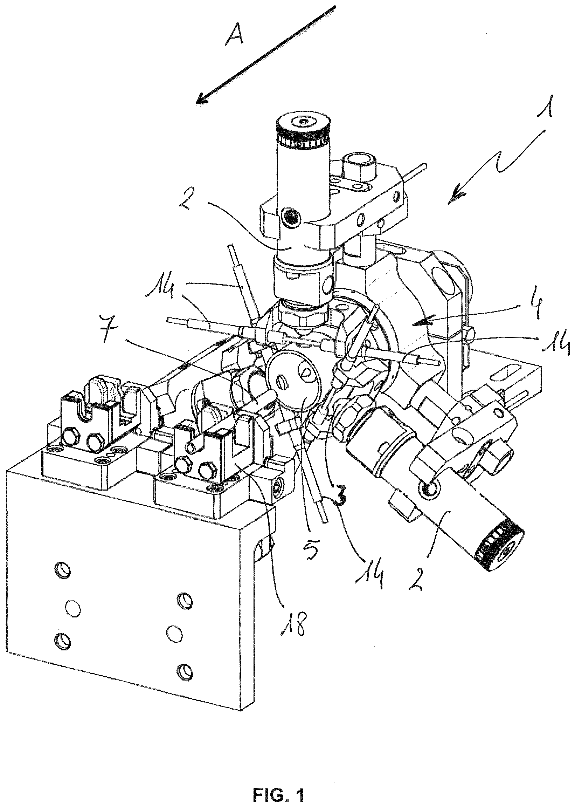

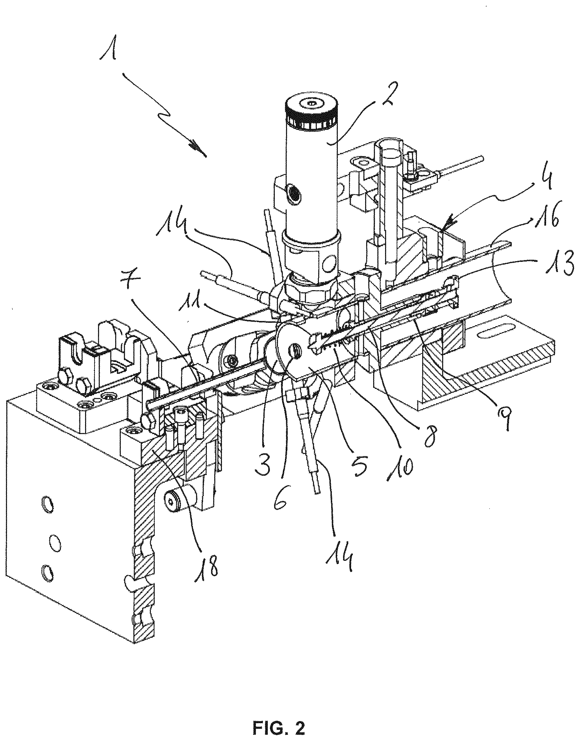

.The opposite end is provided with a plate 13 designed to abut on the flange 9 to react against the preload of the spring so that, in the initial inactive position shown in figures 1 and 2, the enlarged head 11 of the stem 8 is located in a forward position toward the nozzle, or nozzles 3.

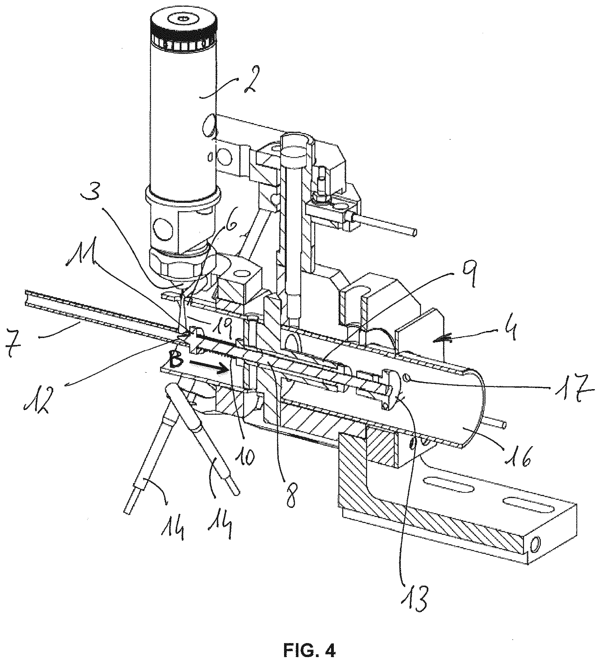

.Advantageously, a suitable position sensor can be mounted on the rear part of the carriage 4 near the plate 13 to detect the correct position of the stem 8 in the operating phase of the work cycle, shown schematically in figure 4. For this purpose, referring to what is illustrated in figure 4, the stem 8 can slide in a cylinder 16 integral with the carriage 4 and provided with a hole 17, through which passes the signal emitted by the sensor; the hole 17 must be suitably positioned so that, when the stem 8 is in the correct operating position, rearward with respect to the nozzle 3 as will be better explained later, the plate 13 is aligned with the axis of the hole 17 so as to intercept the signal and interrupt the circuit; in this manner, the apparatus is informed that the pin 8 positioned correctly to carry out the subsequent operations.

.Suitable means for controlling the effective discharge of the spray of nebulized substance from the nozzle 3 are interposed between the valve, or valves, 2 and the ring 5.

.Such control means can consist of sensors 14 that detect the presence of the nebulized spray, such as for example optical fibres interposed between the ring 5 and the nozzle 3 or photocells supported by brackets 15 of the carriage 4 and arranged in position below and on the sides of the nozzle 3 so that the emitted beam, indicated schematically with the reference numeral 20, is intercepted by the nebulized spray, indicated schematically with reference numeral 19, and the circuit is interrupted. If the circuit remains open, the sensor detects the failed discharge of the spray 19 thus making it possible to carry out the appropriate corrective measures.

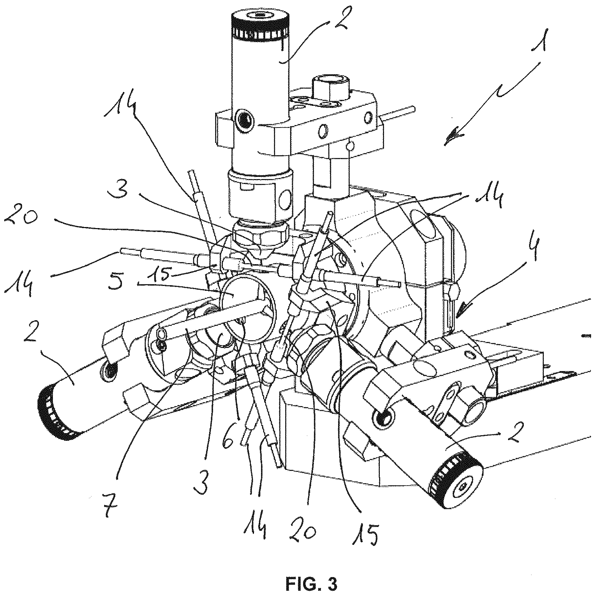

. If more than one valve is present, a sensor 14 will be provided for each valve 2, as exemplified in figure 3.

.The operation of the apparatus is as follows: in the initial position of the operating cycle, illustrated in figures 1 and 2, the hollow body 7 is held in position by a suitable vise 18 and the movable carriage 4 is in a rearward position with respect to the hollow body 7; in this position, the enlarged head 11 of the stem 8 is thrust by the spring means 10 to a forward position with respect to the nozzle, or nozzles 3.

. From this position, the mobile carriage 4 advances toward the hollow body 7 in the direction indicated by the arrow A until the conical head 12 of the stem 8 enters into the mouth of the hollow body 7, centring it the ring 5; a further small forward movement of the carriage 4 brings the enlarged head 11 into abutment with the mouth of the hollow body 7 and pushes the stem 8 backward in the direction shown by the arrow B in figure 4 against the action of the elastic means 10, so as to move the enlarged head 11 backward and bring the end portion of the hollow body 7 into alignment with the nozzle, or nozzles 3.

.If there is a position sensor suitable to generate the signal passing through the hole 17, the forward movement of the carriage 4 is controlled by said sensor and the carriage 4 stops when the plate 13, in its reverse movement, intercepts the signal and thereby interrupts the circuit.

.When the carriage reaches this operating position, shown in figures 3 and 4, in which the mouth of the hollow body 7 is centred by the conical end 12 and closed by the enlarged head 11 of the stem 8, so as to prevent the entrance of any nebulized substance inside the hollow body 7, the nebulized substance, indicated schematically with reference numeral 19, is sprayed through the nozzle, or nozzles 3, of the respective valve 2 and conveyed, through the hole 6 and the ring 5, to the external surface of the end portion of the hollow body 7.

.The discharge of the nebulized spray 19 from the nozzle 3 is controlled by the sensor, or sensors, 14 so that, in the absence of the signal indicating the outflow of the spray, or if the beam 20 is not intercepted by the spray 19, the necessary corrective measures can be adopted.

.When the operation is positively completed, the mobile carriage 4 moves backward and frees the hollow body 17, which can then be transferred toward the next gluing station.

. From the above, it is thus evident how the present invention achieves the initially foreseen purposes and advantages: in fact, the invention devised an apparatus for dosing and controlling a nebulized spray on an elongated hollow body, in particular for medical or food use, capable of resolving the drawbacks of the prior art.

.In particular, the apparatus described above is capable of guaranteeing that the spray of nebulized substance does not contaminate the internal cavity of the hollow body, thanks to the occlusion and centring of the mouth of the hollow body achieved by the stem 8 having the enlarged head 11 and the conical end 12.

.A further advantage is provided by the assurance that the hollow body, such as the tube 7, has effectively received the nebulized substance, thanks to the prearrangement of appropriate sensors 14 suitable to detect the presence of the spray of nebulized substance between the nozzle 3 and the hollow body.

.Naturally, the present invention is receptive to numerous applications, modifications or variants without thereby departing from the scope of patent protection, as defined by claim 1.

.For example, the centring means may consist of an inclined plane or a conical entrance associated with the mobile carriage 4, preferably with the ring 5, and arranged adjacent to and facing said hollow body 7 so that the entry of the hollow body 7 into the ring 5 causes it to be automatically centred therewith.

.Further, as illustrated in figures from 5 to 7 relative to a second embodiment of the apparatus according to the present invention, the occluding means may consist of a pneumatic system that blows a fluid, preferably air, at high pressure inside the hollow body 7 in the direction of the end portion struck by the spray of nebulized substance, so as to prevent the penetration of particles of the nebulized substance through the occluding effect produced by the overpressure created by blowing at the mouth of the hollow body 7. In this manner, the occluding stem sliding inside the mobile carriage 4 can be eliminated as being no longer necessary.

.With reference to figures from 5 to 7, the pneumatic system comprises a fixed body 21 connected with or comprising suitable means, such as a compressor and relative conduits, suitable to generate a flow of air, or other equivalent fluid, at high pressure. The body 21 is arranged facing the carriage 4 and comprises a conduit 22, arranged coaxially with the cylinder 16, from which flows the high pressure fluid to be conveyed toward the hollow body 7.

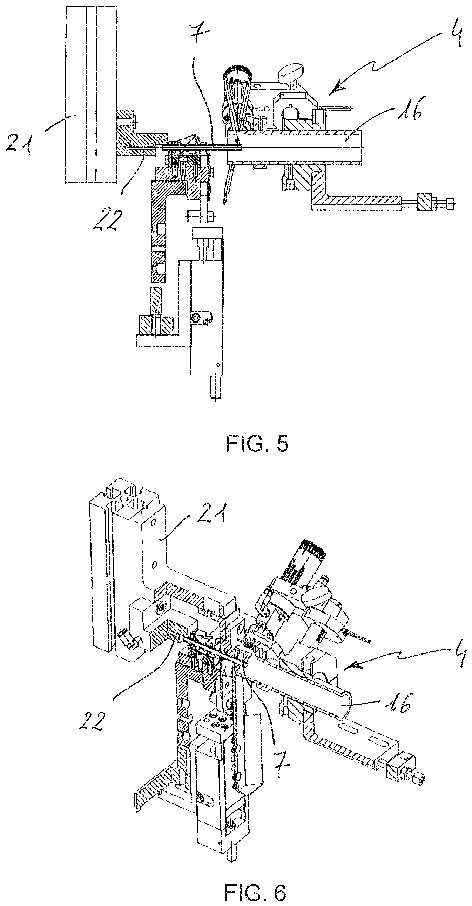

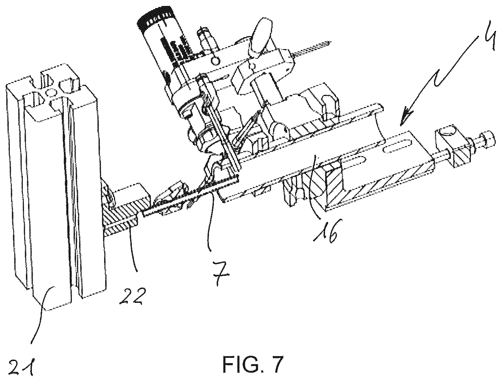

.When the hollow body 7 reaches the dosing and control station, it is coaxially aligned, by suitable centring means described above (not shown in the figures 5 to 7), with the conduit 22 and the cylinder 16. Once the hollow body is in alignment, a highpressure fluid is blown through the conduit 22 so as to strike the end of the hollow body 7 opposite to the one facing the carriage 4, and the nebulized substance is sprayed on the hollow body 7. In this manner, the penetration of particles of nebulized substance inside the hollow body 7 is prevented, thanks to the occluding effect of the overpressure generated by blowing.

.Naturally, the materials and equipment used to implement the present invention, as well as the shapes and dimensions of the individual components, can be the most appropriate for the specific requirements.

## Claims

1. Apparatus for dosing and controlling a nebulized spray on an elongated hollow body, particularly for food or medical use, comprising at least a valve (2) provided with a nozzle (3) adapted to deliver a nebulized substance on said hollow body (7), and a carriage (4) supporting said at least a valve (2), **characterized in that** said carriage (4) comprises a ring (5) provided with at least one hole (6) arranged so as to face said nozzle (3), said carriage (4) being axially movable between an initial inactive position, wherein said carriage (4) is located, in use, at a distance in respect to said hollow body (7), and an operative position wherein, in use, an end portion of said hollow body (7) is substantially aligned with said nozzle (3) to be sprayed by said nebulized spray (19), said apparatus further comprising occluding means (11; 21, 22) and centering means (12), said occluding means (11; 21, 22) and centering means (12) being adapted to cooperate with the mouth of said end portion of said hollow body (7) when said carriage (4) is in said operative position to prevent infiltration of particles of said nebulized substance into the inside of said hollow body (7) and to substantially align said end portion of said hollow body (7) with said nozzle (3), said centering means (12) being integrated in a stem (8) supported by a flange (9) fastened to said carriage (4), said stem (8) being arranged centrally and coaxially to said ring (5) and being configured to slide axially relative to said ring (5) against the action of elastic means (10).

2. Apparatus as in claim 1, wherein said occluding means are formed by an enlarged head (11) arranged at a terminal portion of said stem (8) facing said nozzle (3), said enlarged head (11) being adapted to abut against the mouth of said hollow body (7) when said carriage (4) is in said operative position.

3. Apparatus as in claim 2, wherein said centering means consist of a conical edge (12) formed at said terminal portion of said stem (8) facing said nozzle (3), said conical edge (12) being arranged beyond said enlarged head (11) and adapted to engage the mouth of said hollow body (7) when said carriage (4) is in said operative position.

4. Apparatus as in claim 1, further comprising control means adapted to control the delivery of said nebulized spray (19) from said nozzle (3).

5. Apparatus as in claim 4, wherein said control means consist of sensors (14) interposed between said nozzle (3) and said terminal portion of said hollow body (7), said sensors (14) being adapted to detect said spray (19) of said nebulized substance delivered by said nozzle (3).

6. Apparatus as in claim 1, wherein said occluding means comprise a pneumatic system (21, 22) adapted to blow a fluid at high pressure inside said elongated hollow body (7) towards said end portion adapted to be sprayed by said nebulized spray (19) such that infiltration of particles of said nebulized substance is prevented by the overpressure effect created at the mouth of said hollow body (7).

7. Apparatus as in claim 6, wherein said pneumatic system comprises a fixed body (21) arranged facing said carriage (4) connected to or comprising means suitable to generate a flow of fluid at high pressure, said fixed body (21) comprising a conduit (22) designed to convey said flow toward the end of said hollow body (7) opposite to the end facing said carriage (4).

8. Apparatus as in claim 7, wherein said centering means are adapted to coaxially align said hollow body (7) with respect to said conduit (22) when said highpressure fluid is being blown.

9. Apparatus as in claim 1, wherein said centering means are formed by a conical body or an inclined plane associated to said carriage (4) and arranged adjacent to and facing said hollow body (7).

10. Apparatus as in any of the preceding claims, comprising two or more valves (2) circumferentially arranged around said ring (5) at mutually substantially equidistant angular positions.

11. Production line comprising at least a workstation formed by an apparatus for dosing and controlling a nebulized spray on an elongated hollow body, particularly for food or medical use, as in any of the preceding claims.

## Patentansprüche

1. Vorrichtung zum Dosieren und Steuern eines zerstäubten Sprühnebels auf einem länglichen Hohlkörper, insbesondere für Nahrungsmittel oder medizinische Zwecke, mit mindestens einem Ventil (2) auf dem Hohlkörper (7), das mit einer Düse (3) versehen ist, die ausgebildet ist, eine zerstäubte Substanz auszugeben, und mit einem Schlitten (4), der das mindestens eine Ventil (2) trägt,
**dadurch gekennzeichnet, dass**
der Schlitten (4) einen Ring (5) aufweist, der mit mindestens einem Loch (6) versehen ist, das so angeordnet ist, dass es der Düse (3) zugewandt ist, wobei der Schlitten (4) zwischen einer anfänglichen inaktiven Stellung, in der bei Gebrauch der Schlitten (4) mit einem Abstand in Bezug auf den Hohlkörper (7) angeordnet ist, und einer Betriebsstellung axial bewegbar ist, in der bei Gebrauch ein Endbereich des Hohlkörpers (7) im Wesentlichen zu der Düse (3) ausgerichtet ist, um durch den zerstäubten Sprühnebel (19) besprüht zu werden, wobei die Vorrichtung ferner aufweist: eine Einschlusseinrichtung (11; 21, 22) und eine Zentriereinrichtung (12), wobei die Einschlusseinrichtung (11; 21, 22) und die Zentriereinrichtung (12) ausgebildet sind, mit der Mundöffnung des Endbereichs des Hohlkörpers (7) zusammenzuwirken, wenn der Schlitten (4) sich in der Betriebsstellung befindet, so dass ein Infiltrieren von Teilchen der zerstäubten Substanz in das Innere des Hohlkörpers (7) verhindert wird und der Endbereich des Hohlkörpers (7) im Wesentlichen zu der Düse (3) ausgerichtet wird, wobei die Zentriereinrichtung (12) in einem Stab (8) integriert ist, der von einem Flansch (9) gehalten wird, der an dem Schlitten (4) befestigt ist, wobei der Stab (8) zentral und koaxial zu dem Ring (5) angeordnet und ausgebildet ist, relativ zu dem Ring (5) entgegen die Wirkung einer elastischen Einrichtung (10) axial zu gleiten.

2. Vorrichtung nach Anspruch 1, wobei die Einschlusseinrichtung durch einen vergrößerten Kopf (11) gebildet ist, der an einem Anschlussbereich des Stabs (8) angeordnet ist, der der Düse (3) zugewandt ist, wobei der vergrößerte Kopf (11) geeignet ist, an der Mundöffnung des Hohlkörpers (7) anzuliegen, wenn der Schlitten (4) in der Betriebsstellung ist.

3. Vorrichtung nach Anspruch 2, wobei die Zentriereinrichtung aus einer konischen Kante (12) besteht, die an dem Anschlussbereich des Stabs (8), der der Düse (3) zugewandt ist, ausgebildet ist, wobei die konische Kante (12) über den vergrößerten Kopf (11) hinaus angeordnet und ausgebildet ist, mit der Mundöffnung des Hohlkörpers (7) in Eingriff zu treten, wenn der Schlitten (4) in der Betriebsstellung ist.

4. Vorrichtung nach Anspruch 1, die ferner eine Steuereinrichtung aufweist, die ausgebildet ist, die Ausgabe des zerstäubten Sprühnebels (19) aus der Düse (3) zu steuern.

5. Vorrichtung nach Anspruch 4, wobei die Steuereinrichtung aus Sensoren (14) besteht, die zwischen der Düse (3) und dem Anschlussbereich des Hohlkörpers (7) angeordnet sind, wobei die Sensoren (14) ausgebildet sind, den Sprühnebel (19) der zerstäubten Substanz, die von der Düse (3) ausgegeben wird, zu erfassen.

6. Vorrichtung nach Anspruch 1, wobei die Einschlusseinrichtung ein pneumatisches System (21, 22) aufweist, das ausgebildet ist, ein Fluid mit hohem Druck im Inneren des länglichen Hohlkörpers (7) zu dem Endbereich zu blasen, derart, dass eine Einwirkung auf den zerstäubten Sprühnebel (19) derart erfolgt, dass eine Infiltration von Teilchen der zerstäubten Substanz durch die Überdruckwirkung, die an der Mundöffnung des Hohlkörpers (7) hervorgerufen wird, verhindert wird.

7. Vorrichtung nach Anspruch 6, wobei das pneumatische System einen fixierten Körper (21) aufweist, der so angeordnet ist, dass er dem Schlitten (4) zugewandt ist, und verbunden ist mit oder aufweist eine Einrichtung, die geeignet ist, eine Strömung des Fluids bei hohem Druck zu erzeugen, wobei der fixierte Körper (21) eine Leitung (22) aufweist, die so gestaltet ist, dass sie das Strömen zu dem Ende des Hohlkörpers (7) entgegengesetzt zu dem Ende, das dem Schlitten (4) zugewandt ist, führt.

8. Vorrichtung nach Anspruch 7, wobei die Zentriereinrichtung ausgebildet ist, den Hohlkörper (7) in Bezug auf die Leitung (22) koaxial auszurichten, wenn das Hochdruckfluid eingeblasen wird.

9. Vorrichtung nach Anspruch 1, wobei die Zentriereinrichtung durch einen konischen Körper oder eine geneigte Ebene gebildet ist, der bzw. die mit dem Schlitten (4) verbunden und benachbart zu dem Hohlkörper (7) angeordnet und diesem zugewandt ist.

10. Vorrichtung nach einem der vorhergehenden Ansprüche, mit zwei oder mehr Ventilen (2), die in Umfangsrichtung um den Ring (5) mit zueinander im Wesentlichen gleichen Winkelpositionen angeordnet sind.

11. Fertigungslinie mit mindestens einem Arbeitsplatz, der durch eine Vorrichtung zum Dosieren und Steuern eines zerstäubten Sprühnebels auf einem länglichen Hohlkörper, insbesondere für Nahrung oder medizinische Zwecke, nach einem der vorhergehenden Ansprüche gebildet ist.

## Revendications

1. Dispositif de dosage et de commande d'un spray nébulisé sur un corps creux allongé, en particulier pour usage alimentaire ou médical, comprenant au moins une vanne (2) munie d'une buse (3) apte à délivrer une substance nébulisée sur ledit corps creux (7) et un chariot (4) supportant ladite au moins une vanne (2), **caractérisé en ce que** ledit chariot (4) comprend un anneau (5) prévu avec au moins un trou (6) agencé de sorte à faire face à ladite buse (3), ledit chariot (4) étant mobile axialement entre une position initiale inactive, dans laquelle ledit chariot (4) est situé, en utilisation, à une distance par rapport audit corps creux (7), et une position active dans laquelle, en utilisation, une partie d'extrémité dudit corps creux (7) est sensiblement aligné avec ladite buse (3) destiné à être pulvérisé par ledit spray nébulisé (19), ledit dispositif comprenant en outre des moyens d'occlusion (11 ; 21, 22) et des moyens de centrage (12), lesdits moyens d'occlusion (11 ; 21, 22) et moyens de centrage (12) étant aptes à coopérer avec l'embouchure de ladite partie d'extrémité dudit corps creux (7) lorsque ledit chariot (4) se trouve dans ladite position active pour empêcher l'infiltration de particules de ladite substance nébulisée à l'intérieur dudit corps creux (7), et pour aligner sensiblement ladite partie d'extrémité dudit corps creux (7) avec ladite buse (3), lesdits moyens de centrage (12) étant intégrés dans une tige (8) supportée par une bride (9) attachée audit chariot (4), ladite tige (8) étant disposée centralement et coaxialement audit anneau (5) et étant agencée pour coulisser axialement par rapport audit anneau (5) contre l'action de moyens élastiques (10).

2. Dispositif selon la revendication 1, dans lequel lesdits moyens d'occlusion sont formés par une tête élargie (11) disposée à une partie terminale de ladite tige (8) faisant face à ladite buse (3), ladite tête élargie (11) étant adaptée pour venir en butée contre l'embouchure dudit corps creux (7) lorsque ledit chariot (4) se trouve dans ladite position active.

3. Dispositif selon la revendication 2, dans lequel lesdits moyens de centrage sont constitués d'un bord conique (12) formé à ladite partie terminale de ladite tige (8) faisant face à ladite buse (3), ledit bord conique (12) étant agencé au-delà de ladite tête élargie (11) et adapté pour engager l'embouchure dudit corps creux (7) lorsque ledit chariot (4) se trouve dans ladite position active.

4. Dispositif selon la revendication 1, comprenant en outre un moyen de commande adapté pour commander la fourniture dudit spray nébulisé (19) à partir de ladite buse (3).

5. Dispositif selon la revendication 4, dans lequel lesdits moyens de commande sont constitués de capteurs (14) interposés entre ladite buse (3) et ladite partie terminale dudit corps creux (7), lesdits capteurs (14) étant adaptés pour détecter ledit spray (19) de ladite substance nébulisée délivrée par ladite buse (3).

6. Dispositif selon la revendication 1, dans lequel lesdits moyens d'occlusion comprennent un système pneumatique (21, 22) adapté pour souffler un fluide à haute pression à l'intérieur dudit corps creux (7) allongé en direction de ladite partie d'extrémité apte à être pulvérisé par ledit spray nébulisé (19) de telle sorte que l'infiltration des particules de ladite substance nébulisée est empêchée par l'effet de la surpression créée à l'embouchure dudit corps creux (7).

7. Dispositif selon la revendication 6, dans lequel ledit système pneumatique comprend un corps fixe (21) disposé en regard dudit chariot (4) connecté à ou comprenant des moyens aptes à générer un écoulement de fluide à haute pression, ledit corps fixe (21) comprenant une conduit (22) destiné à transporter ledit écoulement vers l'extrémité dudit corps creux (7) opposée à l'extrémité faisant face audit chariot (4) .

8. Dispositif selon la revendication 7, dans lequel lesdits moyens de centrage sont agencés pour s'aligner de manière coaxiale audit corps creux (7) par rapport audit conduit (22) lorsque ledit fluide à haute pression est soufflé.

9. Dispositif selon la revendication 1, dans lequel lesdits moyens de centrage sont formés par un corps conique ou un plan incliné associé audit chariot (4) et disposés de manière adjacente à et faisant face audit corps creux (7).

10. Dispositif selon l'une quelconque des revendications précédentes, comprenant deux ou plusieurs vannes (2) disposées de manière circonférentielle autour dudit anneau (5) à des positions angulaires mutuellement sensiblement équidistantes.

11. Ligne de production comprenant au moins un poste de travail formé par un dispositif pour le dosage et le contrôle d'un spray nébulisé sur un corps creux allongé, en particulier pour usage alimentaire ou médical, selon l'une quelconque des revendications précédentes.
